(19) Europäisches Patentamt
European Patent Office
Office européen des brevets

(11) **EP 1 490 118 B1**

(12) **EUROPÄISCHE PATENTSCHRIFT**

(45) Veröffentlichungstag und Bekanntmachung des Hinweises auf die Patenterteilung:
**01.09.2010 Patentblatt 2010/35**

(21) Anmeldenummer: **03717221.0**

(22) Anmeldetag: **21.03.2003**

(51) Int Cl.:
*A61L 15/26* $^{(2006.01)}$ *A61L 15/42* $^{(2006.01)}$

(86) Internationale Anmeldenummer:
**PCT/EP2003/002951**

(87) Internationale Veröffentlichungsnummer:
**WO 2003/080133 (02.10.2003 Gazette 2003/40)**

(54) **NARBENREDUKTIONSPFLASTER**

SCAR-REDUCING PLASTER

PANSEMENT REDUCTEUR DE CICATRICES

(84) Benannte Vertragsstaaten:
**AT BE BG CH CY CZ DE DK EE ES FI FR GB GR HU IE IT LI LU MC NL PT RO SE SI SK TR**

(30) Priorität: **22.03.2002 DE 10212866**

(43) Veröffentlichungstag der Anmeldung:
**29.12.2004 Patentblatt 2004/53**

(73) Patentinhaber: **Beiersdorf AG**
**20245 Hamburg (DE)**

(72) Erfinder:
• **KARTHEUS, Holger**
**20255 Hamburg (DE)**
• **HARTKOPF, Carsten**
**22303 Hamburg (DE)**
• **BERG, Thorsten**
**22765 Hamburg (DE)**
• **JÄNICHEN, Jan**
**22149 Hamburg (DE)**
• **KÖHLER, Ulrich**
**22607 Hamburg (DE)**

(56) Entgegenhaltungen:
**EP-A- 0 919 211 WO-A-96/29035**
**US-A- 5 844 013 US-B1- 6 191 216**

**Beschreibung**

Die Erfindung betrifft Narbenreduktionspflaster, die Verfahren zu ihrer Herstellung und deren Verwendung. Die Narbenreduktionspflaster umfassen eine Trägerfolie aus luft- und wasserdampfdurch- und wasserundurchlässiger Polymerschicht, eine Narbenauflage aus einer atmungsaktiven und klebenden Polyurethan-Xerogel-Matrix, wobei die Trägerfolie vollflächig mit der Polyurethan-Matrix beschichtet ist. In einer besonderen Ausführungsform ist eine Randschicht aus der gleichen Polyurethan-Xerogel-Matrix ausgebildet, die sich zum Rand auf eine Dicke von maximal 5 bis 150 $\mu$m abschrägt. Damit wird die Aufrollneigung üblicher Narbenauflagen, verursacht durch Kleidung, Bettwäsche, Hautkontakt oder beim Waschen, stark reduziert. Das wiederum ermöglicht erst die für die Narbenbehandlung lange Tragedauer des Narbenpflasters ohne zusätzliche Fixierabschnitte.

Der Körper reagiert auf eine blutende Hautwunde, indem die Invasion von Krankheitserregern gestoppt und die Blutung gestillt wird. Ist das geschehen, müssen Fremdkörper und Gewebetrümmer entfernt und neues Gewebe aufgebaut werden. Der normale Wundheilungsprozess lässt sich vereinfacht in 4 Phasen unterteilen. Die dabei ablaufenden Prozesse der

    1. Gefäßreaktion und Blutgerinnung,
    2. Entzündung,
    3. Gewebeneubildung (Bildung von Granulationsgewebe und Reepithelisierung) und
    4. Remodellierung

überlappen und bedingen sich zum Teil gegenseitig, so dass die Reihenfolge nur annähernd dem zeitlichen Ablauf der Wundheilung entspricht.
Die Phase der Gefäßreaktion hat die Funktion größere Blutverluste zu verhindern, dabei kommt es zur Vasokonstriktion, die solange anhält, bis die Blutgerinnung die Wunde provisorisch verschließt. In der Entzündungsphase finden vorwiegend katabole, d.h. abbauende Prozesse statt. In dieser Phase der Wundheilung reinigen Granulozyten, Makrophagen und Lymphozyten die Wunde, indem sie körperfremdes Material und Gewebetrümmer aufnehmen und enzymatisch abbauen.
Die Phase der Gewebeneubildung umfasst dagegen reparative, d.h. anabole Reaktionen. Voraussetzung für eine gut heilende Wunde ist eine ausreichende Durchblutung, so dass schon ab dem dritten Tag nach der Verletzung neue Blutgefäße gebildet werden (Angiogenese). Parallel zur Vaskularisierung erfolgt die Bindegewebsneubildung. Entlang des Fibringerüsts wandern Fibroblasten in die Wunde. Diese produzieren die Bindegewebegrundsubstanz, bestehend aus Proteoglykanen und Kollagenfasern, die für die Gewebefestigkeit ausschlaggebend sind.
Im gesunden Gewebe sind die Kollagenfasern nach bestimmten, den Hauptzugrichtungen folgenden Mustern ausgerichtet. Entscheidend für die Kollagensynthese ist das Vorhandensein von Coenzymen und Cofaktoren wie z.B. Ascorbinsäure, Eisen und Kupfer. Besteht ein Mangel an diesen Stoffen, kann die Wundheilung gestört werden. Dabei entsteht Narbengewebe, das durch eine unorganisierte Struktur der Kollagenfasern charakterisiert ist.
Im gleichen Maß, wie das Bindegewebe auswächst, wird das provisorische Fibringerüst abgebaut (Fibrinolyse) und die verschlossenen Gefäße werden wieder rekanalisiert. Mit der Faserneubildung endet die mitotische Aktivität der Fibroblasten. Sie wandeln sich zum einen in Fibrozyten um, zum anderen in Myofibroblasten. Diese enthalten kontraktile Elemente und können sich zusammenziehen. Dabei werden die Kollagenfasern gestrafft und - soweit möglich - gemäß der Hauptzugrichtung des Gewebes ausgerichtet. Als Folge davon zieht sich das funktionstüchtige Hautgewebe am Wundrand zusammen, so daß nur noch ein kleiner Defekt verbleibt.

Die Wundheilung erfordert ein ausbalanciertes Gleichgewicht von gegenläufigen Aktionen wie Zellproliferation und Zellapaptose, Auf- und Abbau von Blutgefäßen sowie dem Auf- und Abbau von Kollagen. Wird dieses Gleichgewicht - besonders beim Auf- und Abbau der Kollagenfasern - in irgendeiner Weise gestört, so kann es zu einer hypotrophen, atrophen oder hypertrophen Narbe führen. Die Ereignisse, die die normale Wundheilung von z.B. hypertropher Wundheilung unterscheiden, beginnen schon während der Entwicklung des Granulationsgewebes. Der sichtbarste Unterschied des Gewebes liegt in der Menge und Orientierung der Kollagenfasern. Bei hypertropher Narbenbildung wird ein Überschuß an Kollagen erzeugt und das Granulationsgewebe zeigt eine Tendenz, die Kollagenfasern in zufälliger und ungeordneter Weise aufzubauen.
Hypertrophe Narben sind gegenüber der umliegenden Haut erhaben und zeigen eine Vielzahl an Variationen in Größe, Form, Farbe und Konsistenz. Diese Kennzeichen hängen zum einen von dem Ort und der Größe der Verletzung und zum anderen von der zeitlichen Entwicklung und der persönlichen Anfälligkeit ab. Die Enden sind normalerweise markant und enden abrupt, manchmal mit fingerförmigen Verlängerungen. Die rötliche Farbe und die Schwellung von frischen Narben stammt von der erhöhten Gefäßdichte. Mit der Zeit strafft sich das Bindegewebe während der Remodelierungsphase und die Blutgefäßdichte bildet sich zurück. Daher sinkt die Narbe etwas ein und verblasst. Der Prozess des Remodelings umfasst den Umbau des Narbengewebes und stellt die am längsten dauernde Phase der Wundheilung dar, er kann sich bis zu 20 Jahre nach der Verwundung hinziehen. Im wesentlichen erfolgt dabei eine Umstrukturierung der Kollagenfasern, wobei sie teilweise durch im Gewebe enthaltene Kollagenasen abgebaut oder aber neu vernetzt werden.

Der allgemeiner Stand der Technik über Verbände und Wundpflaster äußerst sich nicht zu den spezifischen Problemen, die sich bei Behandlung von Narben ergeben. So ist aus der EP 0 264 299 B1 ein Verband bekannt, der aus einem wasserabsorbierenden Abdichtungskissen besteht, das seinerseits von einem oder mehreren Hydrokolloiden gebildet wird. Das oder die Hydrokolloide sind in einem Bindemittel aufgelöst oder mit demselben gemischt.

Das Kissen ist fest von einer wasserdichten Deckungsschicht vollständig umfaßt. Erfindungsgemäß ist das Kissen mindestens um die äußere Peripherie auf eine solche Weise abgeschrägt, dass die Dicke am Rand etwa ein Viertel ihrer maximalen Dicke nicht überschreitet. Die Herstellung erfolgt über ein Druckgussverfahren bei hohen Drücken und hohen Temperaturen. Dieses Verfahren ist für vernetzte Polymergele, beispielsweise Polyurethangele, nicht geeignet.

Konturierte Wundauflagen mit einer Klebmasseschicht bestehend aus quellbaren Hydrokolloiden und wasserunlöslichen, viskosen Bestandteilen, beispielsweise Polyisobutylen, Kautschuk, Silicon oder Polyurethanelastomeren, sind Gegenstand der WO 92/05755. Dabei hat die Klebmasseschicht im Randbereich, die von derselben Art ist wie die Klebmasse im zentralen Bereich, eine Dicke von kleiner 0,5 mm (bevorzugt kleiner 0,3 mm) und eine Breite von mindestens 5 mm (bevorzugt mindestens 10 mm). Die Klebmasse auf Basis von Hydrokolloid zeigt auch auf feuchtem Grund einen Tack. Schaumwundauflagen, wie sie zum Beispiel von der Firma S & N unter dem Namen Cutinova ® thin und Cutinova ® hydro erhältlich sind, sind u. a. beschrieben in DE 42 33 289 A1, in DE 196 18 825 A1 und WO 97/43328.

Der flächige Polyurethanschaum mit einer Dicke von 1 bis 6 mm wird einseitig von einer Polyurethanfolie abgedeckt. Pflaster entsprechender Größe werden aus der Ballenware ausgestanzt. Die so hergestellte Wundauflage entklebt überraschenderweise vollständig bei Aufnahme von Wundflüssigkeit und zeigt dabei nicht die von Hydrokolloiden bekannte Neigung zur Desintegration bei starker Quellung, die dazu führen kann, dass Rückstände des Hydrokolloids in der Wunde verbleiben.

Die ausgestanzten, großflächigen Wundauflagen eignen sich hervorragend zur Versorgung von chronischen oder schwerheilenden Wunden von Patienten, die stationär versorgt werden müssen. Eine positive oder negative Wirkung auf die Narbenbehandlung wird nicht diskutiert. Insbesondere rollt sich das Produkt aufgrund seiner Stanzränder bei mechanischer Belastung leicht auf. Bei Kontakt mit Feuchtigkeit erweisen sich die offenen Stanzränder als Nachteil, da dadurch Wasser an die saugende Schicht gelangen kann und zur Aufquellung und Entklebung des Polyurethanschaums durch seitliches Eindringen von Feuchtigkeit führt.

Weiterhin wird durch die signifikante Produkthöhe (bis zu 4 mm) und den gleichen selbstklebenden Eigenschaften am Rand das Anhaften von Schmutz und ein Aufrollen durch Anhaften von zum Beispiel Kleidungsstücken begünstigt.

Ein Verfahren zur Herstellung eines Verbandes mit dünnen Rändern bestehend aus mindestens zwei Klebmasseschichten wird in EP 0 680 299 A1 beschrieben. Die Klebstoffschichten, die von derselben oder von unterschiedlicher Art sein können, entsprechen einer Anordnung von miteinander verbundenen unterschiedlichen Flächen, die in ihrer Größe zur Spitze abnehmen. Dabei weisen die einzelnen Schichten ein Stufenprofil auf, welches, um einen nach außen kontinuierlichen Verlauf zu erzielen, mit einer weiteren Schicht abgedeckt werden muss. Weiterhin nachteilhaft ist die stufenweise Abflachung des Verbandes auf der hautzugewandten Seite, so dass dadurch an einigen Stellen im Wund- und Wundrandbereich der Kontakt ungleichmäßig erfolgt.

In EP 0 919 211 A2 wird die Herstellung von Wundverbänden mit abgeschrägten Rändern aus thermogeformten Kunststoffträgerfilmen, die trennbeschichtet sind und eine Kavität aufweisen, in die ein selbstklebendes, hydrophiles Polymergel gebracht wird, genannt. Die Verbände weisen eine klebende Deckschicht auf, die wiederum mit einer Schutzschicht abgedeckt ist. Das Verfahren ist aufwendig und für Wundverbände aus "einem Guss" nicht geeignet. Auch hier sind die Verbände auf der wundzugewandten Seite abgeschrägt.

Schließlich seien noch herkömmliche Pflaster zur Versorgung von Wunden genannt (zum Beispiel das Gewebepflaster Hansaplast ® classic der Firma Beiersdorf), die sich nur bedingt für die Anwendung als Narbenpflaster eignen. Nachteilig erweisen sich die geringe Elastizität und die Neigung zum Aufrollen des Trägermaterials an den Kanten des Pflasters bei mechanischer Belastung bei längerer Tragedauer. Zusätzlich wird das Pflaster bei täglicher Körperpflege oder beim Händewaschen stark durchfeuchtet und verliert an Haftvermögen. Herkömmliche Pflaster fallen optisch stark auf, behindern Bewegungen beziehungsweise beeinträchtigen beispielsweise das Tragegefühl im Schuh.

Im Unterschied zu bekannten Wundversorgungsverbänden und Pflastern werden Narbenverbände über einen längeren Zeitraum auf der Haut belassen um einen gewünschten Reduktionseffekt zu gewährleisten. Die übliche Dicke von Wundpflastern von ca. 1 mm und mehr führt bei dieser langen Tragedauer zu einer Ablösung durch Reibung an Kleidung, Bettwäsche, Hautkontakten oder beim täglichen Waschen. Aus diesem Grund sind bekannte Verbände oder Pflaster für den aufgezeigten Zweck nicht einsetzbar. Des weiteren sind an die Narbenauflage ganz andere Anforderungen zu stellen als an Wundauflagen.

Auf Silikongel basierende Narbenabdeckungen, wie sie beispielsweise in EP 782457 A1 beschrieben werden, haben den Nachteil, dass sie wenig atmungsaktiv sind und zu Mazerationseffekten neigen.

Die Mazerationseffekte sind dabei nur ein Nachteil, der sich bei vielen bekannten klebenden Verbänden zeigt. Es besteht bei den Narbenreduktionspflastern einerseits das Problem einer hautschonenden und langanhaltenden Haftung auf der Haut und andererseits einer schmerzfreien, rückstandslosen Ablösung von der Haut. Keine der genannten Offenbarungen liefert dazu einen für den Hersteller als auch für den Anwender akzeptablen Lösungsvorschlag.

In der WO 9629035 und US 5844013 werden lediglich Wundpflaster auf Basis von Polyurethangelen offenbart.

Eine Aufgabe der vorliegenden Erfindung ist es daher die Nachteile des Standes der Technik zu beseitigen. Insbesondere ist es die Aufgabe der vorliegenden Erfindung ein Narbenreduktionspflaster bereit zu stellen, dass eine Rückbildung hyperthroper Narben ermöglicht, eine hautschonende, langanhaltende Klebung gewährleistet, schmerzfrei und rückstandslos ablösbar ist und eine stark verringerte Aufrollneigung an den Kleberändern aufweist um damit eine lange Tragedauer ohne Beeinträchtigung der Narbenreduktion zu erzielen. Darüber hinaus soll ein einfach und kostensparend herzustellendes Narbenpflaster, dass den Stand der Technik bereichert, zur Verfügung gestellt werden.

Gelöst werden die Aufgaben durch die Verwendung eines Pflasters als Narbenreduktionspflaster entsprechend den Hauptansprüchen. Gegenstand der Unteransprüche sind vorteilhafte Ausführungsformen des erfindungsgemäßen Pflasters. Des Weiteren umfasst die Erfindung das Verfahren zu Herstellung von Narbenreduktionspflastern.

Es war überraschend und für den Fachmann nicht vorauszusehen, dass die Verwendung einer Trägerfolie aus luft- und wasserdampfdurch- und wasserundurchlässiger Polymerschicht und einer Narbenauflage aus einer atmungsaktiven und klebenden Polyurethan-Xerogel-Matrix, wobei die Trägerfolie vollflächig mit der Polyurethan-Matrix beschichtet ist, eine Randschicht aus Polyurethan-Xerogel-Matrix ausgebildet ist, die Narbenauflage und die Randschicht aus der gleichen Polyurethan-Xerogel-Matrix bestehen, die Randschicht sich zum Rand auf eine Dicke von maximal 5 bis 150 μm abflacht und die Narbenauflage mittig gelegen stufenlos in die Randschicht übergeht, zur Verfügungstellung eines Narbenreduktionspflasters zur Behandlung hypothroper Narben, den Nachteilen des Standes der Technik abhelfen.

Die Narbenauflage des erfindungsgemäßen Pflasters kann eine Dicke von 0,2 bis 2 mm, insbesondere von 0,5 bis 1,5 mm, aufweisen.

Die Trägerfolie, wie sie aus dem Stand der Technik bekannt ist, besteht aus einer luft- und wasserdampfdurchlässigen aber wasserundurchlässigen Polymerschicht mit einer Dicke von ca. 10 bis 100 μm. Die u.U. flexible Trägerfolie besteht vorzugsweise aus Polymeren von Polyurethan, PE, PP, Polyamid, Polyester oder Polyether-ester.

Xerogele sind Gele, die ihre Flüssigkeit durch verschiedene Behandlungsformen, Verdampfen, Absaugen etc, verloren haben und einen Grenzzustand zu den Festkörpern charakterisieren. Die verwendeten Polyurethan-Xerogele sind daher insbesondere als wasserfrei anzusehen.

Geeignete Polyurethan-Xerogele als Matrix sind Gegenstand der DE 196 18 825, in der hydrophile, selbstklebende Polyurethan-Gele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(III)-Carboxylaten auf Basis von Carbonsäuren mit 2 bis 18 C-Atomen als Katalysatoren sowie
d) Hexamethylendiisocyanat,

mit einem Produkt der Funktionalitäten der Polyurethanbildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.-% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi(III)Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuss von 3 mol dieser Säure mit einem Bi-Gehalt von ca. 17%.

Es werden die Katalysatoren bevorzugt in Mengen von 0,03 bis 0,1 Gew.-%, bezogen auf das Polyol a), eingesetzt.

Als Antioxidantien kommen für die erfindungsgemäßen Polyurethan-Xerogele insbesondere sterisch gehinderte phenolische Stabilisatoren, wie BHT (2,6-Ditert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des $\alpha$-Tocopherol eingesetzt.

Die Antioxidantien werden bevorzugt in Mengen von 0,15 bis 0,5 Gew.-%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethan-Xerogelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$ f_{(Polyol)} \bullet (f_{(Isocyanat)} - 1) \bullet Kennzahl \approx 2 $$

$$ Kennzahl \approx \frac{2}{f_{(Polyol)} \bullet (f_{(Isocyanat)} - 1)} $$

f: Funktionalität der Isocyanat- oder Polyolkomponente

Je nach angestrebter Klebrigkeit oder Elastizität des Gels kann die tatsächlich zu verwendende Isocyanatkennzahl um bis zu + 20% von dem berechneten Wert abweichen. Die erfindungsgemäßen Polyurethan-Xerogelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiter vorzugsweise kommen Polyurethan-Xerogele zum Einsatz, wie sie in der EP 0 665 856 B1 offenbart sind.

Die hydrophilen Polyurethane sind demnach erhältlich aus

1. einem Polyurethan-Xerogel, welches

(A) 25-62 Gew.-%, vorzugsweise 30-60 Gew.-%, besonders bevorzugt 40-57 Gew.-%, bezogen auf die Summe aus (A) und (B), eines kovalent vernetzten Polyurethans als hochmolekulare Matrix und

(B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0 bis 100 Gew.-%, bezogen auf die Summe aus (A) und (B), an Füll- und/oder Zusatzstoffen enthält,

und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschleunigern für die Reaktion zwischen Isocyanat- und Hydroxylgruppen sowie gegebenenfalls
d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen

3 und 6 beträgt und die Isocyanatkennzahl (K) der Formel

$$K = \frac{300 \pm X}{(F_l \bullet F_p) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,
2. einem Wasser absorbierenden Material und/oder
3. einem nichtwässrigen Schäumungsmittel.

Die Polyurethan-Xerogele können aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben werden. Wesentlich ist jedoch, dass bei der Auswahl der gelbbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Die Polyurethan-Xerogel-Matrix kann partiell oder vollflächig geschäumt und/oder ungeschäumt, ungefüllt oder mit zusätzlichen Füllstoffen, wie beispielsweise Superabsorbern, Titandioxid, Zinkoxid, Weichmachern, Farbstoffen etc. eingesetzt werden. Für Anwendungen im Bereich der Narbenreduktion können auch zusätzliche Hydrogele in halbfester bis fester Form mit aktiven Bestandteilen für die zentrale Zone verwendet werden.

Die Polyurethan-Xerogele können gegebenenfalls aus der Polyurethan-Chemie an sich bekannte Zusatzstoffe enthalten, wie zum Beispiel Füllstoffe und Kurzfasern auf anorganischer oder organischer Basis, Metallpigmente, oberflächenaktive Substanzen oder flüssige Streckmittel wie Substanzen mit einem Siedepunkt von über 150 °C.

Als organische Füllstoffe seien beispielsweise Schwerspat, Kreide, Gips, Kieserit, Soda, Titandioxid, Ceroxid, Quarzsand, Kaolin, Russ und Mikrohohlkugeln genannt. An organischen Füllstoffen können zum Beispiel Pulver auf Basis von Polystyrol, Polyvinylchlorid, Harnstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Die Polyurethan-Xerogelschicht ist insbesondere transparent, wasserdampfdurchlässig und klebend. Dies stellt einen signifikanten vorteilhaften Unterschied zu den Silikongel basierenden Narbenauflagen dar. Die Transparenz erhöht zudem die Akzeptanz beim Anwender, da ein Narbenreduktionspflaster üblicherweise über einen längeren Zeitraum auf der Haut getragen wird.

Zur Speicherung von Flüssigkeit kann vorzugsweise ein Superabsorber-Polymer als Pulver eingearbeitet werden.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden und auch Superabsorber der neuen Generation mit nur noch geringen Anteilen an austrockenbarem Wasser und hohem Quellvermögen unter Druck.

Bevorzugte Produkte sind schwach vernetzte Polymerisate auf der Basis Acrylsäure/Natriumacrylat. Solche Natrium-polyacrylate sind als Favor 22-SK (Stockhausen & Co. KG., Deutschland) erhältlich.

Weitere Absorber, zum Beispiel Carboxymethylcellulose und Karaya, sind ebenfalls geeignet.

Es ist daher von Vorteil in die Narbenauflage Superabsorber oder superabsorbierendes Polymer in einer Menge von 0,01 bis 30 Gew.%, insbesondere von 0,5 bis 25 Gew.%, insbesondere 10 Gew.%, bezogen auf die Gesamtmasse der Polyurethan-Xerogel-Matrix, einzuarbeiten.

Polyurethane erweisen sich gegenüber anderen Klebematerialen, wie Polyacrylaten, Kautschuk etc., als äußerst vorteilhaft, da sie kein bekanntes Allergiepotential beinhalten und insbesondere im Gegensatz zu Polyacrylaten nicht hyperallergen wirken.

Die wesentlichen Vorteile der erfindungsgemäßen Verwendung des Narbenreduktionspflasters liegen jedoch in den nachfolgend näher erläuterten unterschiedlichen Wirkmechanismen.

a. Es ist bekannt, dass eine Temperaturerhöhung die Kollagenaseaktivität und damit die Gewebeneubildung fördert. Es ist in verschiedenen Tests nachgewiesen worden, dass bei Aufbringung des erfindungsgemäßen Narbenpflasters eine Temperaturisolierung der Narbe stattfindet. Damit erfolgt eine sensitive Temperaturerhöhung und die Kollagenase wird vorteilhaft stimuliert.

b. Aufgrund des Druckes des Narbenreduktionspflasters auf die Narbenoberfläche wird eine gerichtete Kollagenfaserbildung unterstützt, wodurch die Bildung hyperthroper Narben vermindert bzw. ganz vermieden wird. Das Pflaster wirkt wie die eigene Haut.

c. Aufgrund der hohen Wasserdampfdurchlässigkeit des erfindungsgemäßen Pflasters wird die von der haut abgegebene Feuchtigkeit abgeführt und die darunter liegende Hautschicht nicht aufgeweicht. Es ergibt sich ein hoher Tragekomfort für das erfindungsgemäße Narbenreduktionspflaster, da insbesondere die Tragedauer sich über einen längeren Zeitraum von einigen Tagen bis mehreren Wochen hinziehen kann.

d. Die Klebkraft der eingesetzten Polyurethan-Xerogelmatrix kann derart variiert werden, dass bei Wasserkontakt die Klebkraft verringert bzw. ganz aufgehoben wird. Damit ist einerseits eine hohe Klebkraft während der Tragezeit einstellbar und gleichzeitig eine schmerzfreie und rückstandslose Ablösung nach dem Tragen möglich. Der Anwender braucht beim Entfernen des Pflaster dieses nur unter dem Wasserhahn abzuziehen. Damit ist das erfindungsgemäße Pflaster noch hautschonender, da beim Ablösen die Haut nicht gestresst wird, d.h. die obersten Hautschichten, Corneozyten werden weniger abgezogen.

In den Abbildungen 1 bis 3 sind zur besseren Erläuterung die erfindungsgemäßen Narbenreduktionspflaster skizziert.

Abbildung 1 zeigt die einfachste Ausführungsform, umfassend ein Abdeckmaterial (3) und eine Trägerfolie (1), die vollflächig mit der Polyurethan-Xerogel-Matrix (2) beschichtet ist.

Abbildung 2 zeigt ein Narbenreduktionspflaster, das sich zum Rand (4) hin abschrägt.

Abbildung 3 zeigt ein Narbenreduktionspflaster, das über die gesamte Peripherie eine abgeschrägte Randschicht (4) aufweist.

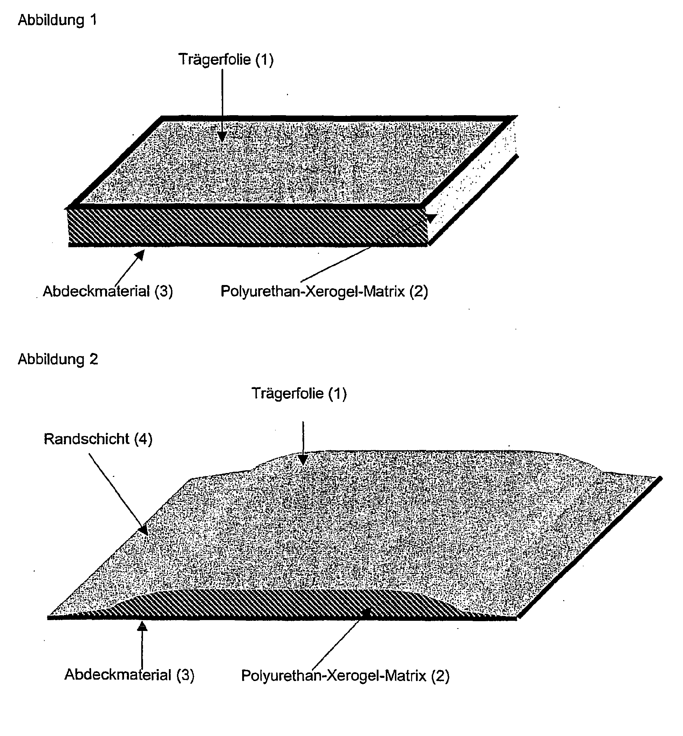

Das erfindungsgemäße Narbenreduktionspflaster kann eine Randschicht aus Polyurethan-Xerogel-Matrix umfassen, wie es in Abbildung 2 dargestellt ist, wobei die Narbenauflage und die Randschicht aus der gleichen Polyurethan-Xerogel-Matrix bestehen und die Randschicht sich zum Rand auf eine Dicke von maximal 5 bis 150 $\mu$m, insbesondere 30 bis 90 $\mu$m, abschrägt. Dies hat den Vorteil, dass das Pflaster die Aufrollneigung, wie sie übliche Pflaster besitzen, verursacht durch Kleidung, Bettwäsche, Hautkontakt oder beim Waschen, stark reduziert. Das wiederum ermöglicht erst die für die Narbenbehandlung lange Tragedauer des Narbenpflasters ohne zusätzliche Fixierungen.

Das Narbenreduktionspflaster kann eine längliche, aufrollbare und beliebig ablängbare Form besitzen. Das hat den Vorteil, dass der Anwender von der Rolle ein entsprechend der individuellen Narbenlänge angepasstes, einstückiges Pflaster zurecht schneiden kann.

Das Pflaster kann zum besseren, sterileren Transport und Lagerung mit einer Schutzabdeckung auf der klebenden Seite ausgestattet sein, die vor dem Anlegen des Pflasters auf die Haut entfernt wird. Diese kann beispielsweise silikonisiertes Papier oder eine insbesondere silikonisierte Folie sein, so dass die klebende Seite während der Lagerung geschützt ist.

Das erfindungsgemäße Pflaster ist als Narbenreduktionspflaster zu verwenden.

Weiterer Vorteil der erfindungsgemäßen Pflaster ist die Herstellung der Narbenauflage mit/ohne Randschicht aus einem Guss. Es ergeben sich stufenlose Übergänge, die eine variable Form und Größe der Pflaster bei der Herstellung erlauben. Damit ist sowohl die Verankerung der Polyurethan-Xerogelschicht auf dem Träger als auch die Haftung auf der Haut verbessert.

So ist die Narbenauflage mittig gelegen und geht stufenlos in die Randschicht über.

Das Verfahren zur Herstellung von Narbenreduktionspflaster verläuft über folgende Stufen, wobei

a.) eine Schicht einer Polyurethan-Xerogel-Matrix auf einen Zwischenträger aufgebracht wird,

b.) das nicht ausgehärtete Polyurethan-Xerogel auf einem Zwischenträger mit einer Trägerfolie insbesondere von einem Rakel zusammenkaschiert wird und

c.) das Laminat anschließend in einen Walzenspalt geführt wird, wo das Polyurethan-Xerogel auf die endgültige Schichtdicke ausgewalzt wird,

d.) die Polyurethan-Xerogelschicht im Randbereich eine Dicke von 5 $\mu$m bis 150 $\mu$m aufweist und

e.) die Narbenauflage aus Polyurethan-Xerogelschicht mittig gelegen stufenlos in die Randschicht übergeht.

Der Walzenspalt kann durch eine Glattwalze und eine Konturenwalze gebildet werden. Oder aber die Konturierung erfolgt nach dem Laminieren mittels einer nachgeschalteten Konturenwalze.

Die Konturenwalze weist Aussparungen auf, die eine Herstellung von gewölbten, zum Rand stufenlos dünner werdenden Pflasterformen gewährleisten.

Durch das Herstellverfahren wird die erfindungsgemäße Herstellung von ein- oder mehrschichtigen, naturgemäß dreidimensional konturierten Produkten ermöglicht. Der Narbenauflagenbereich kann dabei einer herkömmlichen Form entsprechen (eine auf das Trägermaterial aufgebrachte erhabene zum Beispiel rechteckige Wundauflage (siehe Abbildung 1) oder beispielsweise linsenförmig gekrümmt sein, mit nach außen konvexer Form, oder aus kombinierten konvexen und konkaven Elementen, wobei die narbenzugewandte Fläche plan ist.

In der Konturenwalze können verschiedene Einprägungen, Aussparungen vorhanden sein, um eine Formung der Polyurethanschicht zu erzielen. Beispielsweise können diese die Form einer halbkonkaven Linse aufweisen. Dies führt in der Polyurethanschicht zu erhöhten Zentren, die sich zum Rand hin abschrägen. Möglich sind auch Ellipsoide, Quader, Würfel oder andere geometrische Formen, um spezielle Formen in der Polyurethanschicht zu erzeugen.

Der Konturierungsverlauf vom Zentrum zum Rand wird durch die gewählte Form festgelegt, das heißt, die Konturenwalze bestimmt das Design der Kontur.

Die Geometrien der herstellbaren konturierten Formkörper sind vielfältig (rund, elliptisch, quadratisch, dreieckig etc.).

Zur Behandlung, Laminierung der Polyurethan-Xerogelschicht kann im Walzenspalt ein Druck von 1 bar bis 10 bar, insbesondere 5 bar erzeugt werden. Weiterhin sollte der Walzenspalt so dimensioniert sein, dass die Polyurethan-Xerogelschicht eine Dicke von 0,2 bis 2 mm, insbesondere von 0,5 bis 1,5 mm, aufweist.

Erfindungsgemäß erlaubt das Verfahren einen kontinuierlichen Herstellprozess. Damit sind auch Einsparpotentiale bei der Prozessenergie und den Anlageninvestitionen zu generieren.

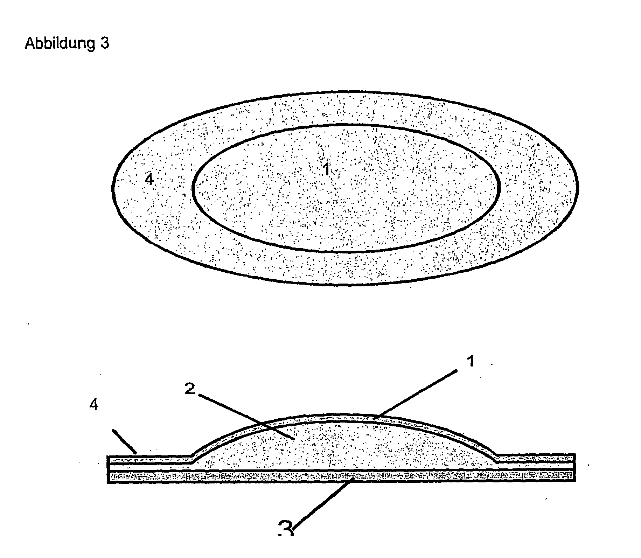

Die dargestellten Ausführungsformen, illustriert durch die Abbildungen 1 bis 3 verdeutlichen die vorliegende Erfindung, ohne sie einzuschränken. Die angegebenen Zahlenwerte bedeuten Gewichtsprozente, bezogen auf das Gesamtgewicht der jeweiligen Zubereitungen sofern nichts anderes angegeben ist.

## Patentansprüche

1.   Verwendung einer Trägerfolie aus luft- und wasserdampfdurch- und wasserundurchlässiger Polymerschicht und einer Narbenauflage aus einer atmungsaktiven und klebenden Polyurethan-Xerogel-Matrix, wobei die Trägerfolie vollflächig mit der Polyurethan-Matrix beschichtet ist, eine Randschicht aus Polyurethan-Xerogel-Matrix ausgebildet ist, die Narbenauflage und die Randschicht aus der gleichen Polyurethan-Xerogel-Matrix bestehen, die Randschicht sich zum Rand auf eine Dicke von maximal 5 bis 150 $\mu$m abflacht und die Narbenauflage mittig gelegen stufenlos in die Randschicht übergeht, zur Verfügungstellung eines Narbenreduktionspflasters zur Behandlung hypothroper Narben.

2.   Verwendung eines Pflasters nach Anspruch 1, **dadurch gekennzeichnet, dass** die Randschicht sich zum Rand auf eine Dicke von 30 bis 90 $\mu$m abflacht.

3.   Verwendung eines Pflasters nach Anspruch 1 oder 2, **dadurch gekennzeichnet, dass** das Pflaster eine längliche, aufrollbare und beliebig ablängbare Form besitzt.

4.   Verwendung eines Pflasters nach Anspruch 1, 2 oder 3, **dadurch gekennzeichnet, dass** die Randschicht um die gesamte Peripherie des Pflasters angeordnet ist,

5.   Verwendung eines Pflasters nach einem der vorstehenden Ansprüche, **dadurch gekennzeichnet, dass** die Trägerfolie aus Polyurethan-, Polyethylen-, Polypropylen-, Polyamid-, Polyester- oder Polyether-ester-Polymeren besteht.

6.   Verwendung eines Pflasters nach einem der vorstehenden Ansprüche, **dadurch gekennzeichnet, dass** das gesamte Pflaster transparent ist.

7.   Verwendung eines Pflasters nach einem der vorstehenden Ansprüche, **dadurch gekennzeichnet, dass** die Polyurethan-Xerogel-Matrix bei Wasserkontakt entklebt.

8.   Verwendung eines Pflasters nach einem der vorstehenden Ansprüche, **dadurch gekennzeichnet, dass** die Narbenauflage eine Dicke von 0,2 bis 2 mm, insbesondere von 0,5 bis 1,5 mm, aufweist

9.   Verwendung eines Pflasters nach einem der vorstehenden Ansprüche, **dadurch gekennzeichnet, dass** in der Narbenauflage superabsorbierendes Polymer in einer Menge von 0.01 bis 30 Gew.%, insbesondere von 0,5 bis 25 Gew.%, insbesondere 10 Gew.%, bezogen auf die Gesamtmasse der Polyurethan-Xerogel-Matrix enthalten ist.

10.  Verwendung eines Pflasters nach einem der vorstehenden Ansprüche, **dadurch gekennzeichnet, dass** die Polyurethan-Xerogel-Matrix partiell oder vollflächig geschäumt und/oder ungeschäumt vorliegt.

11. Verfahren zur Herstellung von Narbenreduktionspflaster, wobei

a.) eine Schicht einer Polyurethan-Xerogel-Matrix auf einen Zwischenträger aufgebracht wird,
b.) das nicht ausgehärtete Polyurethan-Xerogel auf einem Zwischenträger mit einer Trägerfolie insbesondere von einem Rakel zusammenkaschiert wird und
c.) das Laminat anschließend in einen Walzenspalt geführt wird, wo das Polyurethan-Xerogel auf die endgültige Schichtdicke ausgewalzt wird,
d.) die Polyurethan-Xerogelschicht im Randbereich eine Dicke von 5 μm bis 150 μm aufweist und
e.) die Narbenauflage aus Polyurethan-Xerogelschicht mittig gelegen stufenlos in die Randschicht übergeht.

12. Verfahren nach Anspruch 11, **dadurch gekennzeichnet, dass** der Walzenspalt durch eine Glattwalze und eine Konturenwalze gebildet wird.

13. Verfahren nach Anspruch 11, **dadurch gekennzeichnet, dass** nach der Laminierung c.) eine Prägung mittels einer Konturenwalze stattfindet.

14. Verfahren nach Anspruch 12 oder 13, **dadurch gekennzeichnet, dass** die Konturenwalze Aussparrungen aufweist, die eine Herstellung von gewölbten, zum Rand stufenlos abflachenden Pflasterformen gewährleisten.

15. Verfahren nach Anspruch 12 oder 13, **dadurch gekennzeichnet, dass** die Konturenwalze Aussparrungen aufweist, die eine Herstellung beliebiger geometrischer Pflasterformen gewährleisten.

16. Verfahren nach zumindest einem der vorherigen Ansprüche 11 bis 15, **dadurch gekennzeichnet, dass** im Walzenspalt ein Druck von 1 bar bis 10 bar, insbesondere 5 bar erzeugt wird.

17. Verfahren nach zumindest einem der vorherigen Ansprüche 11 bis 16, **dadurch gekennzeichnet, dass** die Polyurethan-Xerogelschicht nach dem Walzenspalt eine Dicke von 0,2 bis 2 mm, insbesondere von 0,5 bis 1.5 mm, aufweist.

18. Verfahren nach einem der Ansprüche 11 bis 17, **dadurch gekennzeichnet, dass** die Polyurethan-Xerogelschicht im Randbereich eine Dicke von 30 bis 90 μm aufweist.

## Claims

1. Use of a backing film composed of air- and water vapor-pervious and water-impervious polymer layer and a scar contact material composed of a breathable and adhesive polyurethane xerogel matrix, where the backing film is coated over the whole area with the polyurethane matrix, an edge layer is formed from polyurethane xerogel matrix, the scar contact material and the edge layer consist of the same polyurethane xerogel matrix, the edge layer flattens out to a thickness not exceeding 5 to 150 μm at the edge, and the scar contact material located in the center transitions continuously into the edge layer, to provide a scar-reducing plaster for the treatment of hypotrophic scars.

2. Use of a plaster according to Claim 1, **characterized in that** the edge layer flattens out to a thickness of from 30 to 90 μm at the edge.

3. Use of a plaster according to Claim 1 or 2, **characterized in that** the plaster has an elongate shape which can be roller up and cut to any length.

4. Use of a plaster according to Claim 1, 2 or 3, **characterized in that** the edge layer is disposed around the entire periphery of the plaster.

5. Use of a plaster according to any of the preceding claims, **characterized in that** the backing film consists of polyurethane, polyethylene, polypropylene, polyamide, polyester or polyether-polyester polymers.

6. Use of a plaster according to any of the preceding claims, **characterized in that** the entire plaster is transparent.

7. Use of a plaster according to any of the preceding claims, **characterized in that** the polyurethane xerogel matrix abheres on contact with water.

8. Use of a plaster according to any of the preceding claims, **characterized in that** the scar contact material has a thickness of from 0.2 to 2 mm, in particular from 0.5 to 1.5 mm.

9. Use of a plaster according to any of the preceding claims, **characterized in that** superabsorbent polymer is present in an amount of from 0.01 to 30% by weight, in particular from 0.5 to 25% by weight, in particular 10% by weight, based on the total mass of the polyurethane xerogel matrix, in the scar contact material.

10. Use of a plaster according to any of the preceding claims, **characterized in that** the polyurethane xerogel matrix is in partly or full-area foamed and/or unfoamed form.

**11.** Process for producing scar-reducing plaster, where

a.) a layer of a polyurethane xerogel matrix is applied to an intermediate backing,
b.) the uncured polyurethane xerogel on an intermediate backing and a backing film are laminated together in particular by a knife, and
c.) the laminate is then fed into a nip where the polyurethane xerogel is rolled out into the final layer thickness,
d.) the polyurethane xerogel layer has a thickness of from 5 $\mu$m to 150 $\mu$m in the edge region, and
e.) the scar contact material consisting of polyurethane xerogel layer located in the center transitions continuously into the edge layer.

**12.** Process according to Claim 11, **characterized in that** the nip is formed by a smooth roll and a contoured roll.

**13.** Process according to Claim 11, **characterized in that** embossing by means of a contoured roll takes place after the lamination c.).

**14.** Process according to Claim 12 or 13, **characterized in that** the contoured roll has recesses which ensure a production of convex plaster shapes sloping steadily to the edge.

**15.** Process according to Claim 12 or 13, **characterized in that** the contoured roll has recesses which ensure a production of any geometric plaster shapes.

**16.** Process according to at least one of the preceding Claims 11 to 15, **characterized in that** a pressure of from 1 bar to 10 bar, in particular 5 bar is generated in the nip.

**17.** Process according to at least one of the preceding Claims 11 to 16, **characterized in that** the polyurethane xerogel layer has a thickness of from 0.2 to 2 mm, in particular of from 0.5 to 1.5 mm, after the nip.

**18.** Process according to any of Claims 11 to 17, **characterized in that** the polyurethane xerogel layer has a thickness of from 30 to 90 $\mu$m in the edge region.

**Revendications**

**1.** Utilisation d'un film de support constitué d'une couche de polymère imperméable à l'eau et perméable à la vapeur d'eau et d'un revêtement cicatriciel à base d'une matrice xérogel-polyuréthanne collante et laissant passer l'air, le film de support étant sur toute sa surface revêtu de la matrice polyuréthanne, une couche périphérique étant constituée de matrice xérogcl-polyuréthanne, le revêtement cicatriciel et la couche périphérique consistant en la même matrice xérogel-polyuréthanne, la couche périphérique s'aplatissant jusqu'au bord en une épaisseur d'au maximum 5 à 150 $\mu$m et le revêtement cicatriciel qui se trouve au milieu se transformant progressivement en la couche périphérique, pour donner un pansement réducteur de cicatrices destiné au traitement de cicatrices hypothropes.

**2.** Utilisation d'un pansement selon la revendication 1, **caractérisée en ce que** la couche périphérique s'aplatit jusqu'au bord en une épaisseur de 30 à 90 $\mu$m.

**3.** Utilisation d'un pansement selon la revendication 1 ou 2, **caractérisée en ce que** le pansement présente une forme allongée, enroulable et pouvant être découpée en longueur de façon quelconque.

**4.** Utilisation d'un pansement selon la revendication 1, 2 ou 3, **caractérisée en ce que** la couche périphérique est disposée sur toute la périphérie du pansement.

**5.** Utilisation d'un pansement selon l'une quelconque des revendications précédentes, **caractérisée en ce que** le film de support consiste en polymères polyuréthanne, polyéthylène, polypropylène, polyamide, polyester ou polyéther-ester.

**6.** Utilisation d'un pansement selon l'une quelconque des revendications précédentes, **caractérisée en ce que** le pansement dans son ensemble est transparent.

**7.** Utilisation d'un pansement selon l'une quelconque des revendications précédentes, **caractérisée en ce que** la matrice xérogel-polyuréthanne se décolle au contact avec l'eau.

**8.** Utilisation d'un pansement selon l'une quelconque des revendications précédentes, **caractérisée en ce que** le revêtement cicatriciel a une épaisseur de 0,2 à 2 mm, en particulier de 0,5 à 1,5 mm.

**9.** Utilisation d'un pansement selon l'une quelconque des revendications précédentes, **caractérisée en ce qu'**un polymère superabsorbant est contenu dans le revêtement cicatriciel en une quantité de 0,01 à 30 % en poids, en particulier de 0,5 à 25 % en poids, plus particulièrement de 10 % en poids, par rapport à la masse totale de la matrice xérogel-polyuréthanne.

**10.** Utilisation d'un pansement selon l'une quelconque des revendications précédentes, **caractérisée en ce que** la matrice xérogel-polyuréthanne est présente non expansée ou expansée sur une partie ou la totalité de la surface.

**11.** Procédé pour la fabrication de pansement réducteur cicatriciel, dans lequel

> a) on applique une couche d'une matrice xérogel-polyuréthanne sur un support intermédiaire,
> b) le xérogel-polyuréthanne non durci sur un support intermédiaire est réuni par stratification, en particulier à l'aide d'une racle, avec un film de support et
> c) le stratifié est ensuite envoyé dans un espace entre des cylindres où le xérogel-polyuréthanne est laminé en l'épaisseur de couche finale,
> d) la couche de xérogel-polyuréthanne présente dans la zone des bords une épaisseur de 5 $\mu$m à 150 $\mu$m et
> e) le revêtement cicatriciel à base de couche de xérogel-polyuréthanne se trouvant au milieu se transforme progressivement en la couche périphérique.

**12.** Procédé selon la revendication 11, **caractérisé en ce que** l'espace entre cylindres est formé par un cylindre lisse et un cylindre gravé.

**13.** Procédé selon la revendication 11, **caractérisé en ce qu'**après la stratification c) a lieu un gaufrage au moyen d'un cylindre gravé.

**14.** Procédé selon la revendication 12 ou 13, **caractérisé en ce que** le cylindre gravé présente des évidements qui assurent une fabrication de formes de pansements bombées, s'aplatissant progressivement vers le bord.

**15.** Procédé selon la revendication 12 ou 13, **caractérisé en ce que** le cylindre gravé présente des évidements qui assurent une fabrication de formes géométriques quelconques de pansements.

**16.** Procédé selon au moins l'une des revendications 11 à 15 précédentes, **caractérisé en ce que** dans l'espace entre cylindres est engendrée une pression de 1 bar à 10 bars, en particulier de 5 bars.

**17.** Procédé selon au moins l'une des revendications 11 à 16, **caractérisé en ce que** la couche de xérogel-polyuréthanne présente après l'espace entre cylindres une épaisseur de 0,2 à 2 mm, en particulier de 0,5 à 1,5 mm.

**18.** Procédé selon l'une quelconque des revendications 11 à 17, **caractérisé en ce que** la couche de xérogel-polyuréthanne présente dans la zone des bords une épaisseur de 30 à 90 $\mu$m.

Abbildung 1

Trägerfolie (1)

Abdeckmaterial (3)          Polyurethan-Xerogel-Matrix (2)

Abbildung 2

Trägerfolie (1)

Randschicht (4)

Abdeckmaterial (3)      Polyurethan-Xerogel-Matrix (2)

Abbildung 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0264299 B1
- WO 9205755 A
- DE 4233289 A1
- DE 19618825 A1
- WO 9743328 A
- EP 0680299 A1
- EP 0919211 A2
- EP 782457 A1

- WO 9629035 A
- US 5844013 A
- DE 19618825
- EP 0665856 B1
- DE 3103499 A1
- DE 3103500 A1
- EP 0147588 A1
- DE 3713601 A1

**In der Beschreibung aufgeführte Nicht-Patentliteratur**

- Polyurethanes, Chemistry and Technology. **Saunders-Frisch.** High Polymers. Interscience Publishers, 1962, vol. XVI, 1, 32-42

- **Becker ; Braun.** Kunststoff- Handbuch, Bd. 7, Polyurethane. Carl-Hauser, 1983, vol. 7, 121 ff